# EUROPEAN PATENT APPLICATION

(11) **EP 1 252 906 A1**
(43) Date of publication of application: **30.10.2002**
(21) Application number: 01500112.6
(22) Date of filing: 26.04.2001
(51) Int. Cl.: A61M 5/28, A61M 5/34, A61M 5/31

(54) **Improved disposable pre-filled syringe**

(71) Applicant: Fada Italia, S.P.A., 00196 Rome (IT)
(72) Inventor: Rotundo, Giovanni, 00196 Rome (IT)
(74) Representative: Del Corral Perales, Laura

(57) **Abstract**

Made up of a cylindrical body (1) containing the medicinal product, which is closed at one of its ends by means of the classic piston (4) for propelling said product to the outside through a mouthpiece (6) and a complementary needle (8). Its characteristics are based on the fact that said cylindrically-shaped mouthpiece (6) houses a valve (10) inside, which keeps the chamber (2) sealed until the syringe is to be used, when said valve is moved axially by the pressure generated in the chamber (2) until the channels (12) in its side wall communicate with the head (7) of the needle (8) and allow the liquid to exit to the outside.

## Description

### OBJECT OF THE INVENTION

The present invention refers to a syringe for clinical use, which is designed to be used once and is the original holder of the pharmacological product to be injected.

The object of the invention is to keep the chamber containing the product hermetically sealed until the latter is injected, and also to ensure at the same time that the complementary hypodermic needle is perfectly fixed to said chamber, specifically to the mouthpiece of the same.

### BACKGROUND OF THE INVENTION

Single-use syringes are known that are constructed from a hollow body with a cylindrical shape, ending at one end in the corresponding mouthpiece where the product exits. Inside said hollow body there is a piston which can reduce or even empty the capacity of said chamber as it advances along the same, during the product injection stage. The complementary hypodermic needle is coupled to said mouthpiece by pressure, aided by a protection cap.

Generally, these syringes are sold empty, sterilised and duly encapsulated, although obviously it is also possible to sell such syringes with the corresponding dose of medicinal product contained in their chamber.

However, in practice this is not viable because the chamber containing the product is not hermetically sealed but open to the outside through the hollow inner part of the needle.

The above problem can apparently be resolved by using a cap with a watertight seal. But this is only a partial solution, since the considerable volume of said cap means that a large-capacity air chamber forms between it and the needle, which is sufficient to have an oxidising or other type of effect on the product contained in the syringe chamber.

### DESCRIPTION OF THE INVENTION

The syringe proposed by the invention resolves the problem described above in a completely satisfactory way, by incorporating a valvular seal in the mouthpiece of the chamber that allows the latter to be kept watertight and, consequently, allows the medicinal product contained inside it to be kept in perfect condition.

Therefore, more specifically, said syringe is made up, in a conventional way, of a cylindrical body ending in a mouthpiece, inside which cylindrical body a classic piston can move axially, with the peculiarity that said mouthpiece, in contrast to the classic conical shape, is cylindrically shaped and a double-seal valve is housed inside it. Said valve is provided with a cylindrical body which ends in a conical point at its inner end, corresponding with which it has large channels in the direction of its generatrices, which extend to a determinate distance from its other end, which is perfectly cylindrical and ends in a flange acting as a check to limit penetration into the mouthpiece of the syringe body, in such a way that said perfectly cylindrical sector determines a perfectly watertight seal on the inner and corresponding surface of the mouthpiece. When the valve moves axially under the effect of the pressure generated in the cylindrical chamber when the piston moves forward, said valve partially emerges from the mouthpiece until it communicates with the head of the needle through its side channels.

In accordance with another feature of the invention, the mouthpiece of the syringe body is housed inside a coaxial casing which has an inner thread and corresponds with another thread in the head of the needle, thus allowing a solid and effective coupling between these elements, which makes it impossible for the needle to move accidentally. Said thread has a free end area in said casing which allows the protective cap of the needle to be coupled inside it by pressure, until the syringe is to be used.

### DESCRIPTION OF THE DRAWINGS

In order to complement the description being given and to promote a better understanding of the features of the invention, in accordance with a preferred example of a practical embodiment of the same, said description is accompanied by a set of drawings which form an integral part of the same, in which, by way of illustration and non-restrictively, the following are represented:
Figure 1 shows a diagrammatic representation in side elevation of a disposable pre-filled syringe in accordance with the object of the present invention.
Figure 2 shows a side elevation in diametric cross-section view of the body of the syringe of the foregoing figure.
Figure 3 shows an enlarged detail of the foregoing figure, representing the mouthpiece in which the valve is implanted.
Figure 4 shows a side elevation view of said valve.
Figure 5 shows, finally, an axial view of the valve of the foregoing figure.

### PREFERRED EMBODIMENT OF THE INVENTION

In the light of the above-mentioned drawings, it may be observed that the syringe proposed by the invention is made up, as is conventional, of a cylindrical body (1) which forms a chamber (2) containing the medicinal product in question, which is open at its rear end through which it receives a rod (3). Said rod ends at one end in the piston (4) which is perfectly fitted to the inner wall of the body (1) and pushes the product contained in the chamber (2) when a plunger (5) situated on its outer end is operated upon. Said body (1) ends in a mouthpiece (6) for coupling the head (7) of a hypodermic needle (8), which is protected by a cap (9).

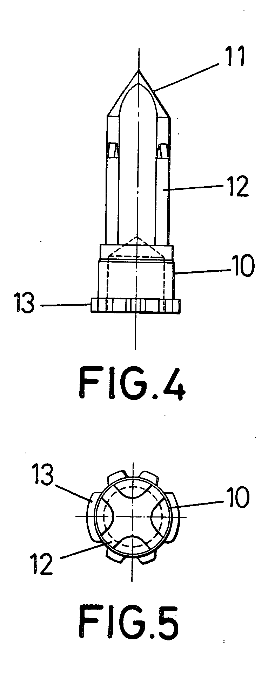

On the basis of this basic and conventional structure, the invention is centred on the fact that the mouthpiece (6), in contrast to the classic truncoconical shape, has a cylindrical shape and receives a valve inside it in the form of a cylindrical body (10), preferably hollow and open at one end, to facilitate its construction process. The closed end (11) of said cylindrical body is conical in shape to facilitate the passage of the pressure generated inside the chamber (2) through a plurality of channels (12) running in the direction of its generatrices, equiangularly distributed on the periphery of said body (10), affecting most of the area of the same, while the rest of said body, corresponding to the reference (10), is perfectly cylindrical and ends in a flange (13) which, as mentioned above, acts as a check to limit the penetration of the valve inside the mouthpiece (6), acting on the entrance (14) of the same. Said flange (13), as can be observed especially in figure 5, has a plurality of asymmetrical cut-outs distributed on its periphery.

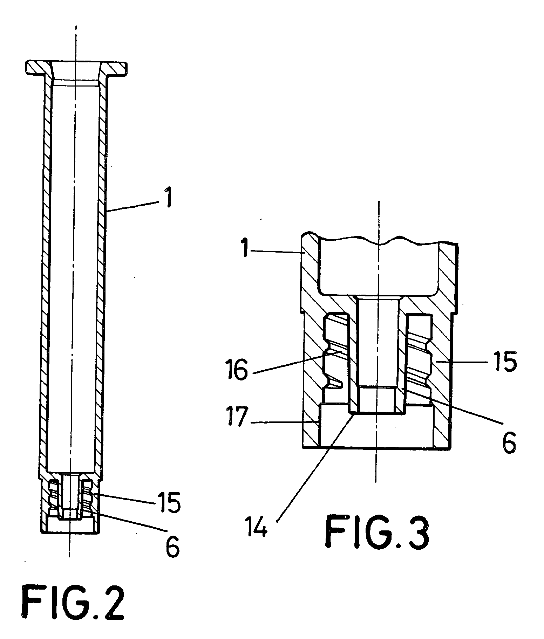

The mouthpiece (6), as can be observed especially in figure 3, is housed inside a casing (15), which is coaxial to the same and also an axial extension of the body (1). Said casing (15) is over-sized axially with respect to the mouthpiece (6), so that in the first section it incorporates an inner thread (16) for receiving the head (7) of the needle, while it receives the protective cap (9) by pressure in its free end area (17).

In accordance with this structure, the operation of the syringe is as follows:

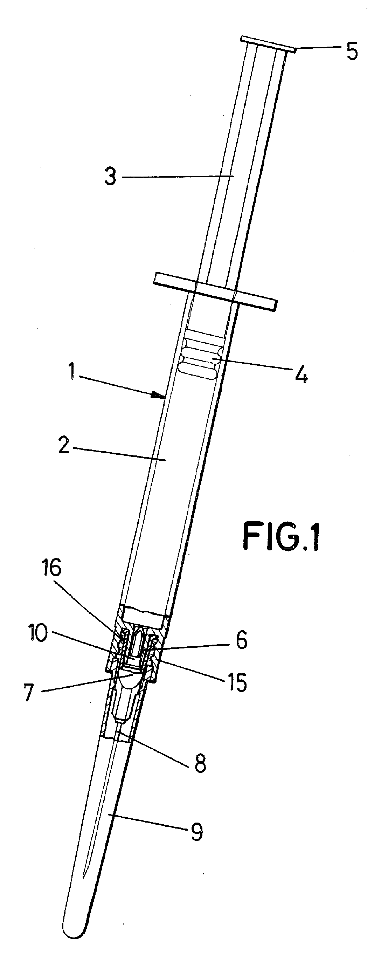

Prior to the syringe's being used, the piston (4) remains retracted, in the position shown in figure 1, the chamber (2) is occupied by the medicinal product in question, and said chamber is hermetically sealed at one of its ends and by means of the piston (4), and at its other end by means of the valve (10), the hypodermic needle (8) being coupled to the casing (15) of the body (1) and protected by the cap (9).

When the syringe is to be used, it is sufficient to remove said cap (9) and press on the plunger (5) of the rod (3) to generate an excess of pressure inside the chamber (2), thus causing the valve (10) to move axially until it reaches a limiting situation in which the channels (12) of said valve pass beyond the mouthpiece (6). At this point direct communication is established between the chamber (2) and the head of the needle and, through the latter, with the outside. The medicine can now be injected, as with any conventional syringe. During the injection operation, the needle (8) remains solidly joined to the body (1) thanks to the threaded coupling of its head (7) with the casing (15).

## Claims

1. A disposable pre-filled syringe of the type that are made up of a cylindrical body which receives an operating rod in one of its ends, with its corresponding piston, while its other end ends in a mouthpiece for coupling the head of a hypodermic needle and, optionally, for also coupling a protective cap for said needle, **characterised in that** said mouthpiece (6) which axially ends the body (1) is cylindrical in shape and houses a valve (10) inside in the form of a cylindrical body, which ends in its inner end in a conical point. Said cylindrical body is provided, in a large area close to said conical point (11), with a number of channels (12), running in the direction of its generatrices, which are interrupted before they reach the opposite end of the valve, in which there is a perfectly cylindrical area (10), ending in a flange (13) in its outer part. Said flange acts as a check to limit the penetration of the valve inside the mouthpiece (6), when it rests on the entrance (14) of the latter, so that said valve constitutes a sealing plug which keeps the chamber (2) of the syringe containing the medicinal product watertight until said syringe is to be used, when the pressure generated in said chamber by the piston (4) causes the valve (10) to move axially until it reaches its open position.

2. A disposable pre-filled syringe in accordance with claim 1, **characterised in that** the cylindrical mouthpiece (6) is housed inside a casing (15) that is coaxial to the mouthpiece (6) and is an extension of the side wall of the body (1), provided with an inner threaded area (16) in its bottom area for coupling, by means of this system, with the head (7) of the needle (8), which is correspondingly threaded; said casing also has a smooth end area (17) for coupling the protective cap (9) of the needle by pressure.
